# EUROPEAN PATENT APPLICATION

(11) **EP 2 502 550 A2**
(43) Date of publication of application: **26.09.2012**
(21) Application number: 12160923.4
(22) Date of filing: 23.03.2012
(51) Int. Cl.: A61B 1/00, A61B 1/018, A61B 1/05

(54) **Swing-out surgical camera**

(30) Priority: 24.03.2011 US 201161466979 P; 07.02.2012 US 201213367410
(71) Applicant: Tyco Healthcare Group LP, Mansfield, MA 02048 (US)
(72) Inventor: Viola, Frank, Sandy Hook, CT Connecticut 06482 (US)
(74) Representative: Soames, Candida Jane

(57) **Abstract**

A surgical camera assembly includes an elongated tubular member and a surgical camera. The elongated tubular member includes a lumen extending therethrough that is configured to permit passage of a surgical instrument. The surgical camera is rotatably coupled to the elongated tubular member towards the distal end thereof and is rotatable relative thereto between a closed position and an open position. The surgical camera is rotated from the closed position to the open position upon passage of the surgical instrument through the lumen of the elongated tubular member and includes a proximal surface configured to facilitate translation of the surgical camera from the closed position to the open position upon contact of the surgical instrument with the proximal surface of the surgical camera within the lumen of the elongated tubular member.

## Description

### CROSS-REFERENCE TO RELATED APPLICATION

The present application claims priority to, and benefit of, U.S. Provisional Patent Application No. 61/466,979 entitled "Swing-Out Surgical Camera" filed on March 24, 2011, the entire contents of which are hereby incorporated by reference herein.

### BACKGROUND

### Technical Field

The present disclosure relates to surgical instruments, and more particularly, to a swing-out camera attachable to surgical instrumentation for providing better visualization within an internal surgical site.

### Background of Related Art

Endoscopic surgical procedures are minimally invasive procedures in which operations are carried out within the body by using elongated instruments inserted through small entrance openings in the body. Minimally invasive procedures are desirable in that they allow for quicker recovery time and shorter hospital stays as compared to open surgical procedures. Minimally invasive procedures also leave minimal scarring (both internally and externally) and reduce patient discomfort during the recovery period. However, because the interior dimensions of the entrance openings into the body are necessarily small, only elongated, small diametered instrumentation may be used to access the internal surgical site.

During a typical minimally-invasive surgical procedure, a surgical camera, or endoscope, is inserted through an access opening in the body to permit the surgeon to view the internal site. As can be appreciated, not only do these cameras require an additional access opening in addition to those created for the insertion of other surgical instrumentation, but they may also require manipulating and/or repositioning each time the other surgical instrumentation is manipulated within the internal surgical site and/or each time a new instrument is introduced into the surgical site. Thus, a minimally-invasive surgical camera that does not require a separate access opening and that is capable of moving in conjunction with the other surgical instrumentation within the internal surgical site is desirable.

### SUMMARY

In accordance with one embodiment of the present disclosure, a surgical camera assembly is provided. The surgical camera assembly includes an elongated tubular member and a surgical camera. The elongated tubular member defines a longitudinal axis and includes a lumen extending longitudinally therethrough. The lumen of the elongated tubular member is configured to permit passage of a surgical instrument therethrough. The surgical camera is rotatably coupled to the elongated tubular member. Specifically, the surgical camera is rotatable between a closed position, wherein the surgical camera is substantially disposed within the lumen of the elongated tubular member, and an open position, wherein the surgical camera is substantially displaced from the lumen of the elongated tubular member. The surgical camera is rotated from the closed position to the open position upon passage of the surgical instrument through the lumen of the elongated tubular member. In particular, the surgical camera includes a proximal surface configured to facilitate translation of the surgical camera from the closed position toward the open position upon contact of the surgical instrument with the proximal surface of the surgical camera within the lumen of the elongated tubular member.

In one embodiment, the surgical camera is biased toward the closed position.

In another embodiment, the proximal surface of the surgical camera defines a helical-shaped configuration.

In still another embodiment, the surgical camera assembly further includes one or more cleaning elements. The cleaning element(s) is configured to clean a lens of the surgical camera upon movement of the surgical camera between the closed position and the open position, e.g., the cleaning element may be configured to wipe clean the lens of the camera as the camera is rotated between the closed position and the open position.

In yet another embodiment, the elongated tubular member includes a seal member disposed within the lumen thereof. The seal member is configured to inhibit the escape of fluid through the lumen of the elongated tubular member. The seal member may be a zero-closure seal member, i.e., to create a fluid-tight seal in the absence of a surgical instrument inserted through the lumen of the elongated tubular member, and/or may be configured to create a seal about a surgical instrument inserted through the lumen of the elongated tubular member. Further, multiple seals may be provided, e.g., both a zero-closure seal and an instrument seal.

In still yet another embodiment, the surgical camera is hingably coupled to the elongated tubular member via one or more hinge joints. In such an embodiment, the surgical camera is rotatable about the hinge(s) between the open and closed positions.

Another embodiment of a surgical camera assembly is also provided in accordance with the present disclosure. In this embodiment, the surgical camera assembly includes an elongated tubular member defining a longitudinal axis and having a lumen extending longitudinally therethrough. The elongated tubular member is configured to permit passage of a surgical instrument therethrough. A capsule is coupled to the elongated tubular member at a distal end thereof and is longitudinally translatable relative to the elongated tubular member between an initial position, wherein the capsule is disposed in abutting relation with the distal end of the elongated tubular member, and an extended position, wherein the capsule is spaced-apart from the elongated tubular member. The capsule is configured to dock on the surgical instrument upon passage of the surgical instrument through the lumen of the elongated tubular member and at least partially through a lumen defined through the capsule such that the capsule is longitudinally translated in conjunction with longitudinal translation of the surgical instrument. A surgical camera is rotatably coupled to the capsule. Specifically, the surgical camera is rotatable between a closed position, wherein the surgical camera is substantially disposed within the lumen of the capsule, and an open position, wherein the surgical camera is substantially displaced from the lumen of the capsule. The surgical camera is translated in conjunction with the capsule between the initial position and the extended position.

In one embodiment, the capsule is biased toward the retracted position. Further, the surgical camera may be configured similarly as in any of the embodiments above.

In another embodiment, the capsule includes a pick-up element disposed at a distal end thereof and extending inwardly into the lumen of the capsule. The pick-up element is configured to dock the capsule on the surgical instrument upon passage of the surgical instrument at least partially through the lumen of the capsule.

In still another embodiment, the capsule is releasably coupled to the elongated tubular member.

### BRIEF DESCRIPTION OF THE DRAWINGS

Various embodiments of the subject instrument are described herein with reference to the drawings wherein:

Fig. 1 is side, perspective view of a surgical camera assembly provided in accordance with one embodiment of the present disclosure, wherein a surgical camera of the surgical camera assembly is camera disposed in an initial position;

Fig. 2 is a side, perspective view of the surgical camera assembly of Fig. 1, wherein the surgical camera is disposed in an open position;

Fig. 3 is a side, perspective view of the surgical camera assembly of Fig. 1, wherein the surgical camera is disposed in an open and extended position;

Fig. 4 is a side, cross-sectional view of the surgical camera assembly of Fig. 1 inserted through an opening in tissue;

Fig. 5 is a side, cross-sectional view of the surgical camera assembly of Fig. 1 inserted through an opening in tissue with a surgical instrument inserted therethrough and with the surgical camera in the open position;

Fig. 6 is a side, cross-sectional view of the surgical camera assembly of Fig. 1 inserted through an opening in tissue with the surgical instrument inserted further therethrough and with the surgical camera in the open and extended position;

Fig. 7 is a side, perspective view of another embodiment of a surgical camera assembly provided in accordance with the present disclosure, wherein a surgical camera of the surgical camera assembly is camera disposed in an initial position;

Fig. 8 is a side, perspective view of the surgical camera assembly of Fig. 7, wherein the surgical camera is disposed in an open position;

Fig. 9A is a longitudinal, cross-sectional view of the surgical camera assembly of Fig. 7, wherein the surgical camera is disposed in the initial position; and

Fig. 9B is a longitudinal, cross-sectional view of the surgical camera assembly of Fig. 7, wherein a surgical instrument inserted through the surgical camera assembly is urging the surgical camera from the initial position to the open position.

### DETAILED DESCRIPTION

Embodiments of the present disclosure are described in detail with reference to the drawing figures wherein like reference numerals identify similar or identical elements. As used herein, the term "distal" refers to the portion that is being described which is further from a user, while the term "proximal" refers to the portion that is being described which is closer to a user.

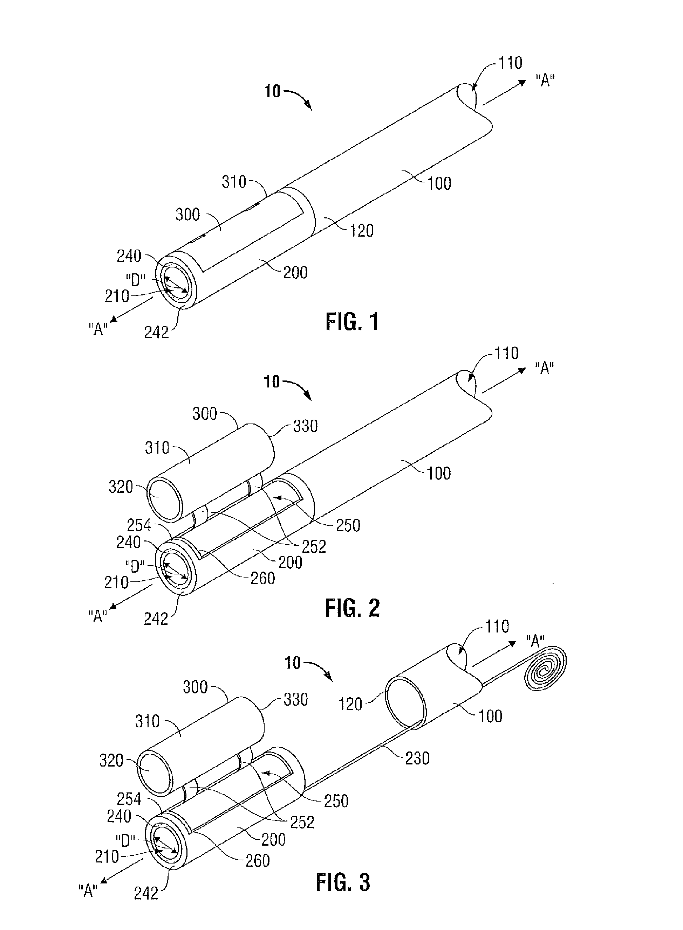

Referring now to Figs. 1-3, a surgical camera assembly provided in accordance with the present disclosure is shown generally identified by reference numeral 10. Surgical camera assembly 10 includes an elongated tubular member, or cannula 100 defining a longitudinal axis "A-A" and having a lumen 110 extending longitudinally therethrough, a capsule 200 coupled to elongated tubular member 100 at a distal end 120 thereof and having a lumen 210 extending longitudinally therethrough that is substantially aligned with lumen 110 of elongated tubular member 100, and a surgical camera 300 coupled to the capsule 200. As will be described in greater detail below, surgical camera 300 is rotatably mounted on capsule 200 such that surgical camera 300 is rotatable relative to capsule 200 between a closed position (Fig. 1), wherein surgical camera 300 is substantially disposed within lumen 210 of capsule 200, i.e., such that surgical camera 300 does not extend beyond the dimensions of capsule 200, and an open position (Figs. 2-3), wherein surgical camera 300 is substantially spaced-apart from lumen 210 of capsule 200, i.e., such that surgical camera 300 is displaced from and positioned adjacent to capsule 200. Further, both capsule 200 and surgical camera 300, which is coupled thereto, are longitudinally translatable relative to elongated tubular member 100 between a retracted position (Figs. 1-2), wherein capsule 200 is disposed in abutting relation with distal end 120 of elongated tubular member 100, and an extended position (Fig. 3), wherein capsule 200 and surgical camera 300 are longitudinally displaced, i.e., longitudinally spaced-apart, from distal end 120 of elongated tubular member 100.

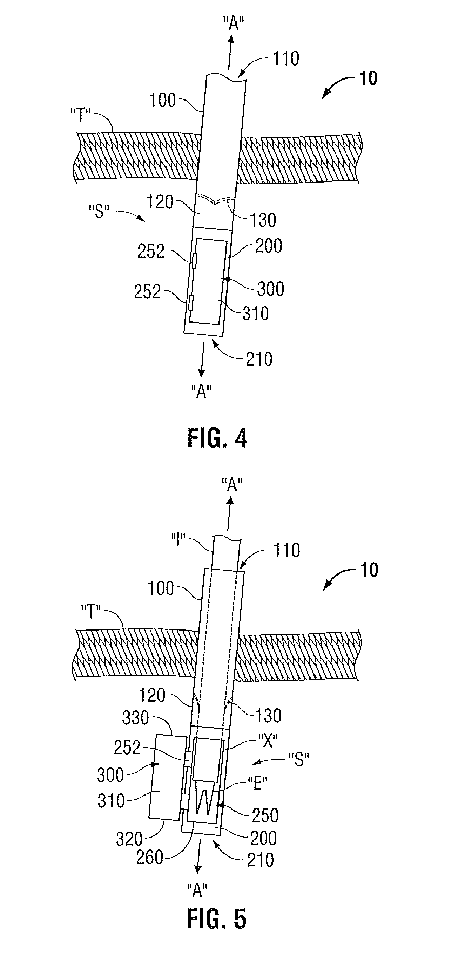

Continuing with reference to Figs. 1-3, elongated tubular member 100 is formed from any suitable bio-compatible material and is dimensioned for insertion through an opening in tissue "T," e.g., through a surgical access port (not shown), an incision in tissue, or a natural body orifice, and for positioning adjacent an internal surgical site "S" (see Figs. 5-6). Lumen 110 of elongated tubular member 100 defines a diameter sufficient to permit passage of surgical instrumentation, e.g., surgical instrument "I," therethrough and into the internal surgical site "S" (see Figs. 5-6). Further, as shown in Figs. 4-6, elongated tubular member 100 may also include a seal member 130 disposed within lumen 110. Seal member 130 may be configured as a zero-closure seal, i.e., such that a substantially fluid-tight seal is created in the absence of surgical instrumentation inserted through lumen 110, and/or may be configured as an instrument seal, i.e., to create a substantially fluid-tight seal about surgical instrumentation inserted through lumen 110. Alternatively, multiple seal members 130 may be provided, e.g., a zero-closure seal and an instrument seal. As will be described in detail below, elongated tubular member 100 may be specifically configured for use with surgical camera assembly 10, or may simply be any suitable elongated tubular member, or cannula 100 configured to engage capsule 200 and surgical camera 300 thereon. In other words, elongated tubular member 100 may be integrally associated with capsule 200 and surgical camera 300, or capsule 200 and surgical camera 300 may be independent of any particular elongated tubular member 100, thus allowing replacement and/or transfer of capsule 200 and surgical camera 300 between suitable elongated tubular members 100.

As shown in Figs. 1-3, and as mentioned above, capsule 200 is coupled to elongated tubular member 100 at distal end 120 thereof. Capsule 200 defines a generally tubular configuration having a lumen 210 extending longitudinally therethrough and may define a diameter similar to that of elongated tubular member 100 so as to act as an extension thereof, i.e., such that lumens 110 and 210 are substantially aligned with one another (i.e. coaxial) to permit insertion of surgical instrumentation through lumen 110 of elongated tubular member 100 and into lumen 210 of capsule 200.

With continued reference to Figs. 1-3, capsule 200 is movably coupled to elongated tubular member 100. More specifically, capsule 200 is coupled to elongated tubular member 100 via one or more flat springs 230 that permit longitudinal extension and retraction of capsule 200 relative to elongated tubular member 100, while biasing capsule 200 towards the retracted position. However, it is also envisioned that capsule 200 be coupled to elongated tubular member 100 via any other suitable mechanism that permits extension and retraction of capsule 200. Further, it is envisioned that flat spring 230 be removably coupled to elongated tubular member 100, e.g., via a releasable latch mechanism (not shown) or any other suitable mechanism, such that capsule 200 and flat spring 230 may be replaced and/or transferred to other suitable elongated tubular members, or cannulas 100.

In the retracted position, as shown in Figs. 1 and 2, capsule 200 is disposed in abutting relation with distal end 120 of elongated tubular member. In this position, flat spring 230 is in an at-rest position, biasing capsule 200 toward abutment with distal end 120 of elongated tubular member 100. Capsule 200 further includes a docking element 240 disposed at distal end 242 thereof. Docking element 240 extends radially inwardly from capsule 200 into lumen 210 thereof to define a reduced internal diameter "D." As such, upon entry of a surgical instrument "I" (Figs. 5-6) into with the reduced-diametered portion of lumen 210, or upon contact of the surgical instrument "I" (Figs. 5-6) with docking element 240, capsule 200 is "docked" on the distal end of the surgical instrument "I" (Figs. 5-6). For example, as will be described in detail below with reference to Figs. 5-6, the reduced diametered portion of lumen 210, created by docking element 240, permits the end effector "E" of surgical instrument "I" to pass therethrough, but inhibits passage of the shaft "X" of surgical instrument "I" therethrough. This configuration "docks" capsule 200 on the distal end of the shaft "X" of the surgical instrument "I" such that, upon further distal translation of surgical instrument "I," the distal end of the shaft "X" of surgical instrument "I" urges docking element 240 and, thus, capsule 200, distally. More particularly, this further distal translation of surgical instrument "I" effects similar distal translation of the "docked" capsule 200 relative to elongated tubular member 100 and against the bias of flat spring 230 from the retracted position (Figs. 1-2) to the extended position (Fig. 3).

Referring still to Figs. 1-3, capsule 200, as mentioned above, includes surgical camera 300 coupled thereto. Surgical camera 300 is rotatably coupled to capsule 200 and is movable relative to capsule 200 between an closed position (Fig. 1), wherein surgical camera 300 is substantially disposed within lumen 210 of capsule 200, and an open position (Figs. 2-3), wherein surgical camera 300 is rotated to a position adjacent capsule such that surgical camera 300 is substantially spaced-apart from lumen 210 of capsule 200. More particularly, Capsule 200 defines a window 250 through a lateral outer-peripheral surface thereof to permit surgical camera 300 to rotate between the open and closed positions. A pair of hinges 252 interconnect surgical camera 300 and capsule 200 to permit rotation of surgical camera 300, although any other suitable mechanism (not shown) capable of permitting rotation of surgical camera 300 between the open and closed positions may also be provided. Further, capsule 200 may include one or more cleaning elements 260, e.g., a brush or other suitable cleaning element, disposed on a distal rim 254 of window 250 that is configured to wipe clean distal lens 320 surgical camera 300 as surgical camera 300 is moved between the open and closed positions. Such a feature, as can be appreciated, helps eliminate any tissue, fluids and/or other debris that may have accumulated on lens 320 surgical camera 300 during insertion of surgical camera assembly 10 into an internal surgical site "S" (see Figs. 4-6).

Surgical camera 300, as best shown in Figs. 2-3, includes an outer housing 310, which houses the video imaging and other electrical components (not explicitly shown) of surgical camera 300, and a distal lens 320 that is aligned substantially parallel to the longitudinal axis "A-A" of elongated tubular member 100. Surgical camera 300 may be configured as a wireless surgical camera, or may include wiring (not explicitly shown) that extends through or along elongated tubular member 100, ultimately coupling surgical camera 300 to an external display (not shown). Additionally, surgical camera 300, or hinges 252 thereof may be removably coupled to capsule 200 to permit use of different types of cameras 300 with surgical camera assembly 10. Surgical camera 300 may also include an illumination source (not explicitly shown) for illuminating the viewing area within the internal surgical site "S" (See Figs. 4-6).

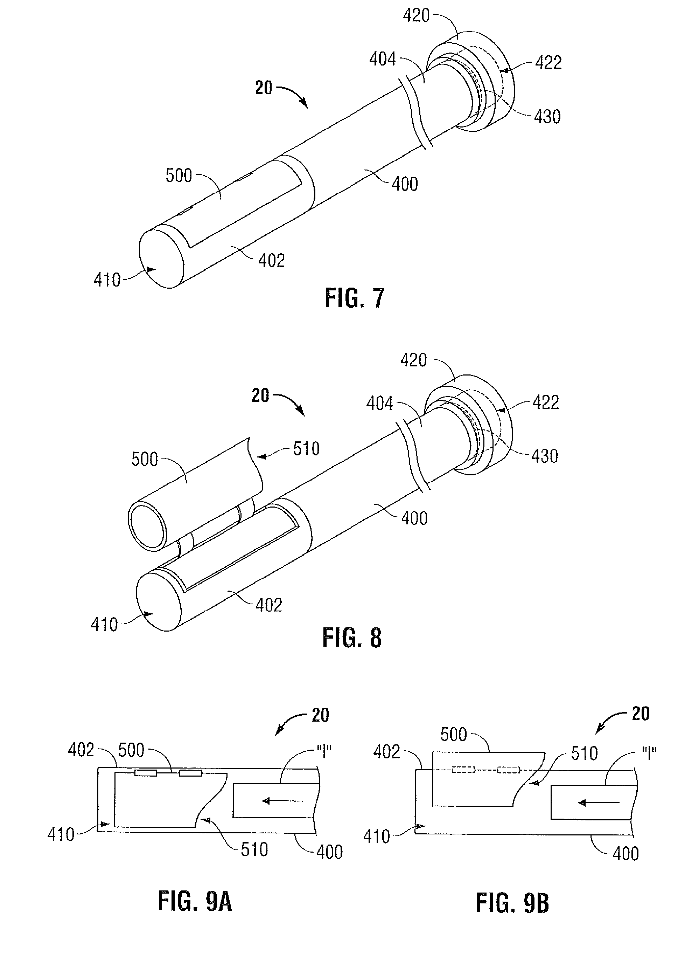

With reference again to Figs. 1-3, surgical camera 300 may be biased toward the closed position where, as shown in Fig. 1, the outer periphery of camera housing 310 of surgical camera 300 is substantially flush with the outer peripheral surface of capsule 200 to facilitate insertion of surgical camera assembly 10 into an internal surgical site "S" (Figs. 4-6) and to inhibit the possibility of catching, or snagging of surgical camera assembly 10. Further, surgical camera 300 may be configured to move from the closed position to the open position upon passage of a surgical instrument "I" (Figs. 5-6) into lumen 210 of capsule 200. More specifically, upon passage of a surgical instrument "I" (Figs. 5-6) into lumen 210 of capsule 200, the distal end of the surgical instrument "I" (Figs. 5-6) contacts surgical camera 300, urging surgical camera 300 to rotate from the closed position (Fig. 1) to the open position (Fig. 2), thus allowing the surgical instrument "I" (Figs. 5-6) to pass further through lumen 210 of capsule 200. Outer housing 310 of surgical camera 300 may define a sloped, angled, or helical proximal surface 330 to facilitate transitioning of surgical camera 300 from the closed position to the open position, as will be described in greater detail below with reference to Figs. 9A-9B. As can be appreciated, upon removal of surgical instrument "I" (Figs. 5-6) from lumen 210 of capsule 200, surgical camera 300 is returned, under the bias, to the closed position.

Turning now to Figs. 4-6, the use and operation of surgical camera assembly 10 will be described. Initially, in embodiments where capsule 200 of surgical camera assembly 10 is replaceable, surgical camera assembly 10 is assembled. More specifically, an appropriate capsule 200, including surgical camera 300, is engaged to an elongated tubular member 100. The particular elongated tubular member 100, e.g., the dimensions or configuration thereof, selected may depend on the surgical procedure to be performed, the surgical instrumentation to be inserted therethrough, anatomical considerations, or other factors. Further, the configuration of capsule 200 and/or surgical camera 300 may likewise be chosen in accordance with these or other factors. Once surgical camera 300 is engaged to capsule 200, and once capsule 200 is engaged to elongated tubular member 100, surgical camera assembly 10 is ready for use.

In use, as shown in Fig. 4, with surgical camera 300 disposed in the closed position within lumen 210 of capsule 200 and with capsule 200 disposed in the retracted position in substantial abutment with distal end 120 of elongated tubular member 100, surgical camera assembly 10 is inserted though an opening in tissue "T" and is positioned adjacent an internal surgical site "S." As can be appreciated, in the closed and retracted position, surgical camera assembly 10 defines a compact configuration to facilitate insertion into and manipulation within the internal surgical site "S." Further, in the closed position, surgical camera 300 is still operable, allowing the surgeon to visualize the internal surgical site "S," e.g., distally along the longitudinal axis "A-A" of elongated tubular member 100, to assist in the positioning of surgical camera assembly 10 therein. With surgical camera assembly 10 positioned within the internal surgical site, seal member 130 maintains a substantially fluid-tight seal, thus inhibiting the escape (or entry) of fluids from the internal surgical site "S."

Turning now to Figs. 4-5, once surgical camera assembly 10 is positioned as desired within the internal surgical site "S," a surgical instrument, e.g., surgical instrument "I," may be inserted through lumen 110 of elongated tubular member 100. As shown in Fig. 5, surgical instrument "I" includes an end effector assembly "E" having a pair of jaw members for grasping tissue therebetween, although it is envisioned that any suitable surgical instrument "I" having an end effector assembly "E" operable to perform a surgical function within the internal surgical site "S" may be inserted through lumen 110 of elongated tubular member 100. As surgical instrument "I" is inserted distally through lumen 110 of elongated tubular member 100, surgical instrument "I" passes through seal member 130, which sealingly engages surgical instrument "I" to maintain the substantially fluid-tight seal of the internal surgical site "S."

With continued reference to Figs. 4-5, upon further translation of surgical instrument "I" through lumen 110 of elongated tubular member 100, surgical instrument "I" is eventually advanced into contact with camera housing 310 of surgical camera 300, which is initially disposed in the closed position within lumen 210 of capsule 200. As surgical instrument "I" is urged into contact with proximal surface 330 of surgical camera 300, surgical camera 300 is swung-out, or rotated our of lumen 210 of capsule 200 from the closed position to the open position. The positioning of surgical instrument "I" within lumen 210 of capsule inhibits surgical camera 300 from returning to the closed position while surgical instrument "I" is positioned therein. As mentioned above, and as will be described in greater detail below (see Figs. 9A-9B), proximal surface 330 of surgical camera 300 may be sloped or angled to facilitate transitioning of surgical camera 300 from the closed position to the open position upon urging by surgical instrument "I." Further, upon rotation of surgical camera 300 from the closed position to the open position, also as mentioned above, cleaning element 260 wipes fluids and/or debris from lens 320 of surgical camera 300 as surgical camera 300 passes through window 250 of capsule 200 to help ensure adequate visualization through lens 320 of surgical camera 300. In this open position of surgical camera 300, the direction of view is substantially parallel to longitudinal axis "A-A," thus allowing the surgeon to view end effector assembly "E" of surgical instrument "I" the area adjacent to and surrounding end effector assembly "E."

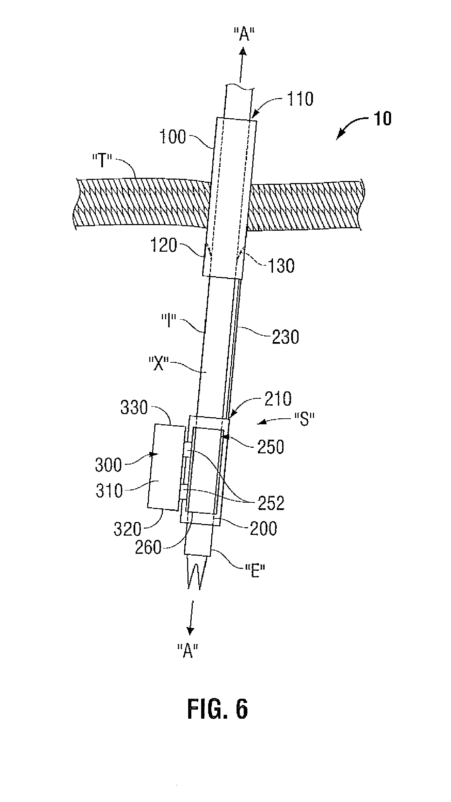

With surgical camera 300 displaced from lumen 210 of capsule 200, as shown in Fig. 5, surgical instrument "I" may be advanced further distally along longitudinal axis "A-A" and through the now vacated lumen 210 of capsule 200. It should be noted that, at this point, capsule 200 remains disposed in the retracted position, wherein capsule 200 is disposed in abutting relation with distal end 120 of elongated tubular member 100. As surgical instrument "I" is translated further through lumen 210 of capsule 200, end effector assembly "E" of surgical instrument "I" eventually passes through docking element 240 (Figs. 1-3) and emerges from distal end 242 (Figs. 1-3) of capsule 200. However, although end effector assembly "E" of surgical instrument "I" is capable of passing through docking element 240 (Figs. 1-3) of capsule 200, shaft "X" of surgical instrument "I" defines a greater diameter than the reduced-diameter portion "D" (Figs. 1-3) of lumen 210 of capsule 200 created by docking element 240 (Figs. 1-3). Thus, rather than shaft "X" of surgical instrument "I" simply passing through docking element 240 (Figs. 1-3), docking element 240 (Figs. 1-3) of capsule 200 is docked on the distal end of shaft "X" of surgical instrument "I," with end effector assembly "E" extending distally therefrom as best shown in Fig. 6.

Referring to Fig. 6, with capsule 200 docked on the distal end of shaft "X" of surgical instrument "I," further distal translation of surgical instrument "I" through lumen 110 of elongated tubular member 100 similarly translates capsule 200 distally relative to elongated tubular member 100 from the retracted position toward the extended position against the bias of flat spring 230. As can be appreciated, and as shown in Fig. 6, this configuration maintains surgical camera 300 "docked" in position adjacent end effector assembly "E" of surgical instrument "I" as surgical instrument "I" is advanced further into the internal surgical site "S," thus providing the surgeon with a relatively close-up view of the end effector assembly "E" and the area adjacent to and surrounding end effector assembly "E", regardless of the depth of insertion of surgical instrument "I" relative to elongated tubular member 100. Accordingly, the surgeon can perform a surgical task within the internal surgical site "S" using end effector assembly "E" and aided by the visualization provided by surgical camera 300, without requiring repositioning of the surgical camera 300 each time surgical instrument "I" is maneuvered and/or manipulated within the internal surgical site "S."

Upon completion of the surgical procedure, surgical instrument "I" may be translated proximally and removed from lumen 210 of capsule 200. As surgical instrument "I" is translated proximally, capsule 200 is similarly translated proximally under the bias of flat sprint 230 from the extended position back towards the retracted position. As surgical instrument "I" is translated further proximally, such that surgical instrument "I" is no longer disposed within lumen 210 of capsule 200, surgical camera 300 is returned under its bias back to the closed position within lumen 210 of capsule 200. Ultimately, upon removal of surgical instrument "I" from lumen 110 of elongated tubular member 100, and with surgical camera 300 disposed in the closed position and capsule 200 disposed in the retracted position, surgical camera assembly 10 may be removed from the surgical site "S."

Turning now to Figs. 7-9B, another embodiment of a surgical camera assembly 20 is shown. Surgical camera assembly 20 is similar to surgical camera assembly 10 (Figs. 1-6) and generally includes an elongated tubular member 400 having a lumen 410 extending longitudinally therethrough. However, elongated tubular member 410 of surgical camera assembly 20 includes a base 420 disposed at proximal end 404 thereof. Base 420 defines a lumen 422 extending longitudinally therethrough in communication with lumen 410 of shaft 400. Base 420 further includes a seal member 430, similar to seal member 130 (Figs. 4-6), that is disposed within lumen 422 and is configured to create a substantially fluid-tight seal in the absence of surgical instrumentation inserted therethrough and/or to create a substantially fluid-tight seal about surgical instrumentation inserted therethrough.

With continued reference to Figs. 7-9B, surgical camera 500 is rotatably coupled to elongated tubular member 400 towards distal end 402 thereof. More specifically, surgical camera 500 is rotatably mounted on elongated tubular member 400 such that surgical camera 500 is rotatable relative to elongated tubular member 400 between a closed position (Fig. 7), wherein surgical camera 500 is substantially disposed within lumen 410 of elongated tubular member 400, and an open position (Fig. 8), wherein surgical camera 500 is substantially spaced-apart from lumen 410 of elongated tubular member 400. Thus, surgical camera assembly 20 includes a surgical camera 500 that is coupled directly to elongated tubular member 400 and is rotatable relative thereto, as compared to surgical camera assembly 10, wherein surgical camera 300 is coupled to capsule 200 and is rotatable and longitudinally translatable relative to elongated tubular member 100 (see Figs. 1-6). In other words, in the embodiment of Figs. 7-9B, surgical camera 500 is not longitudinally extendable relative to elongated tubular member 400, but is longitudinally fixed on elongated tubular member 400. Surgical camera assembly 20 is otherwise similar to surgical camera assembly 10 (Figs. 1-6), except for any additional differences mentioned below. Accordingly, surgical camera 500 and elongated tubular member 400 may include any of the features of surgical camera 300 and elongated tubular member 100, respectively, discussed above with regard to surgical camera assembly 10 (See Figs. 1-6) that are consistent with the use and operation of surgical camera assembly 20, as will be described below.

Turning now to Figs. 9A and 9B, in conjunction with Figs. 7-8, the use and operation of surgical camera assembly 20 will be described. Initially, surgical camera 500 is disposed in the closed position within lumen 410 of elongated tubular member 400 such that surgical camera assembly 20 may be inserted though an opening in tissue "T" (Figs. 4-6) and into position adjacent an internal surgical site "S" (Figs. 4-6). Once surgical camera assembly 20 is positioned as desired, a surgical instrument "I," may be inserted through lumen 422 of base 420, seal member 430, and into lumen 410 of elongated tubular member 400, as shown in Fig. 9A. As surgical instrument "I" is inserted distally further through lumen 410 of elongated tubular member 400, surgical instrument "I" eventually contacts helical proximal surface 510 of surgical camera 500, although other surface configurations, e.g., curved or angled, are contemplated.

With continued reference to Figs. 9A-9B, upon further translation of surgical instrument "I" through lumen 410 of elongated tubular member 400, surgical instrument "I" is urged into helical proximal surface 510 of surgical camera 500, causing surgical camera 500 to begin to rotate from the closed position (Fig. 7), toward the open position (Fig. 8), due to the helical-shaped proximal surface 510 of surgical camera 500. Further, due to this helical-shaped configuration of proximal surface 510, surgical camera 500 is continuously urged to rotate to the open position as surgical instrument "I" is advanced through lumen 410 of tubular member 400, i.e., surgical instrument "I" urges surgical camera 500 toward the open position regardless of the rotational orientation of proximal surface 510 of surgical camera 500 due to the helical-shaped configuration thereof. Similarly as described above with reference to surgical camera assembly 10 (Figs. 1-6), the positioning of surgical instrument "I" within lumen 410 of elongated tubular member 400 inhibits surgical camera 500 from returning to the closed position while surgical instrument "I" is positioned therein.

With surgical camera 500 disposed in the open position, surgical instrument "I" may be advanced further distally through lumen 410 of elongated tubular member 400 and into position to perform a surgical task within the internal surgical site. As can be appreciated, the surgical task is aided by the visualization provided by surgical camera 500, which provides visualization of the area adjacent to and surrounding surgical instrument "I."

Upon completion of the surgical procedure, surgical instrument "I" may be translated proximally and removed from lumen 410 of elongated tubular member 400. As surgical instrument "I" is translated proximally, surgical camera 500 is returned under its bias back to the closed position within lumen 410 of elongated tubular member 400. Thereafter, surgical camera assembly 20 may be removed from the surgical site.

From the foregoing and with reference to the various figure drawings, those skilled in the art will appreciate that certain modifications can also be made to the present disclosure without departing from the scope of the same. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.
The invention may be described by reference to the following numbered paragraphs:-
1. A surgical camera assembly, comprising:
   an elongated tubular member defining a longitudinal axis and having a lumen extending longitudinally therethrough, the elongated tubular member configured to permit passage of a surgical instrument therethrough;
   a surgical camera rotatably coupled to the elongated tubular member towards a distal end thereof, the surgical camera rotatable between a closed position, wherein the surgical camera is substantially disposed within the lumen of the elongated tubular member, and an open position, wherein the surgical camera is substantially displaced from the lumen of the elongated tubular member, the surgical camera including a proximal surface configured to facilitate translation of the surgical camera from the closed position toward the open position upon contact of the surgical instrument with the proximal surface of the surgical camera within the lumen of the elongated tubular member.
2. The surgical camera assembly according to paragraph 1, wherein the surgical camera is biased toward the closed position.
3. The surgical camera assembly according to paragraph 1, wherein the proximal surface of the surgical camera defines a helical-shaped configuration.
4. The surgical camera assembly according to paragraph 1, further comprising at least one cleaning element, the at least one cleaning element configured to clean a lens of the surgical camera upon movement of the surgical camera from the closed position to the open position.
5. The surgical camera assembly according to paragraph 1, further comprising a seal member disposed within the lumen of the elongated tubular member, the seal member configured to inhibit the escape of fluid through the lumen of the elongated tubular member.
6. The surgical camera assembly according to paragraph 1, wherein the surgical camera is hingably coupled to the elongated tubular member via at least one hinge, the surgical camera rotatable about the at least one hinge between the open and closed positions.
7. A surgical camera assembly, comprising:
   an elongated tubular member defining a longitudinal axis and having a lumen extending longitudinally therethrough, the elongated tubular member configured to permit passage of a surgical instrument therethrough;
   a capsule coupled to the elongated tubular member at a distal end thereof, the capsule longitudinally translatable relative to the elongated tubular member between an initial position, wherein the capsule is disposed in abutting relation with the distal end of the elongated tubular member, and an extended position, wherein the capsule is spaced-apart from the elongated tubular member, the capsule configured to dock on the surgical instrument upon passage of the surgical instrument through the lumen of the elongated tubular member and at least partially through a lumen defined through the capsule such that the capsule is longitudinally translated in conjunction with longitudinal translation of the surgical instrument; and
   a surgical camera rotatably coupled to the capsule, the surgical camera rotatable between a closed position, wherein the surgical camera is substantially disposed within the lumen of the capsule, and an open position, wherein the surgical camera is substantially displaced from the lumen of the capsule, the surgical camera translating in conjunction with the capsule between the initial position and the extended position.
8. The surgical camera assembly according to paragraph 7, wherein the surgical camera is biased toward the closed position.
9. The surgical camera assembly according to paragraph 7, wherein the capsule is biased toward the retracted position.
10. The surgical camera assembly according to paragraph 7, wherein the surgical camera is rotated from the closed position to the open position upon passage of the surgical instrument from the lumen of the elongated tubular member into the lumen of the capsule.
11. The surgical camera assembly according to paragraph 7, wherein the capsule includes a pick-up element disposed at a distal end thereof and extending inwardly into the lumen of the capsule to dock the capsule on the surgical instrument upon passage of the surgical instrument at least partially through the lumen of the capsule.
12. The surgical camera assembly according to paragraph 7, wherein the capsule further includes at least one cleaning element, the at least one cleaning element configured to clean a lens of the surgical camera upon movement of the surgical camera from the closed position to the open position.
13. The surgical camera assembly according to paragraph 7, further comprising a seal member disposed within the lumen of the elongated tubular member, the seal member configured to inhibit the escape of fluid through the lumen of the elongated tubular member.
14. The surgical camera assembly according to paragraph 7, wherein the surgical camera is hingably coupled to the capsule via at least one hinge, the surgical camera rotatable about the at least one hinge between the open and closed positions.
15. The surgical camera assembly according to paragraph 7, wherein the capsule is releasably coupled to the elongated tubular member.
16. A method of providing visualization within an internal surgical site, comprising the steps of:
   providing a surgical camera assembly including an elongated tubular member having a lumen extending longitudinally therethrough and a surgical camera rotatably coupled to the elongated tubular member towards a distal end thereof;
   inserting the surgical camera assembly through an opening in tissue such that the distal end of the elongated tubular member is disposed within the internal surgical site; inserting a surgical instrument through the lumen of the elongated tubular member; and translating the surgical instrument distally through the lumen of the elongated tubular member such that the surgical instrument contacts a proximal surface of the surgical camera, urging the surgical camera to rotate from a closed position, wherein the surgical camera is substantially disposed within the lumen of the elongated tubular member, to an open position, wherein the surgical camera is substantially displaced from the lumen of the elongated tubular member to permit passage of the surgical instrument therethrough.
17. The method according to paragraph 16, wherein the proximal surface of the surgical camera defines a helical-shaped configuration to facilitate rotation of the surgical camera from the closed position to the open position.
18. The method according to paragraph 16, further comprising the step of remocing the surgical instrument from the lumen of the elongated tubular member such that the surgical camera is returned to the closed position.
19. The method according to paragraph 16, further comprising a capsule coupled to the elongated tubular member at a distal end thereof, the capsule including a lumen extending therethrough.
20. The method according to paragraph 19, further comprising the steps of:
   translating the surgical instrument distally to extend into the lumen of the capsule such that the capsule is docked on the surgical instrument; and
   translating the surgical instrument further distally such that the capsule is similarly translated distally relative to the elongated tubular member from a retracted position to an extended position.

## Claims

1. A surgical camera assembly, comprising:
an elongated tubular member defining a longitudinal axis and having a lumen extending longitudinally therethrough, the elongated tubular member configured to permit passage of a surgical instrument therethrough;
a surgical camera rotatably coupled to the elongated tubular member towards a distal end thereof, the surgical camera rotatable between a closed position, wherein the surgical camera is substantially disposed within the lumen of the elongated tubular member, and an open position, wherein the surgical camera is substantially displaced from the lumen of the elongated tubular member, the surgical camera including a proximal surface configured to facilitate translation of the surgical camera from the closed position toward the open position upon contact of the surgical instrument with the proximal surface of the surgical camera within the lumen of the elongated tubular member.

2. The surgical camera assembly according to claim 1, wherein the surgical camera is biased toward the closed position.

3. The surgical camera assembly according to claim 1 or claim 2, wherein the proximal surface of the surgical camera defines a helical-shaped configuration.

4. The surgical camera assembly according to any preceding claim, further comprising at least one cleaning element, the at least one cleaning element configured to clean a lens of the surgical camera upon movement of the surgical camera from the closed position to the open position.

5. The surgical camera assembly according to any preceding claim, further comprising a seal member disposed within the lumen of the elongated tubular member, the seal member configured to inhibit the escape of fluid through the lumen of the elongated tubular member.

6. The surgical camera assembly according to any preceding claim, wherein the surgical camera is hingably coupled to the elongated tubular member via at least one hinge, the surgical camera rotatable about the at least one hinge between the open and closed positions.

7. A surgical camera assembly, comprising:
an elongated tubular member defining a longitudinal axis and having a lumen extending longitudinally therethrough, the elongated tubular member configured to permit passage of a surgical instrument therethrough;
a capsule coupled to the elongated tubular member at a distal end thereof, the capsule longitudinally translatable relative to the elongated tubular member between an initial position, wherein the capsule is disposed in abutting relation with the distal end of the elongated tubular member, and an extended position, wherein the capsule is spaced-apart from the elongated tubular member, the capsule configured to dock on the surgical instrument upon passage of the surgical instrument through the lumen of the elongated tubular member and at least partially through a lumen defined through the capsule such that the capsule is longitudinally translated in conjunction with longitudinal translation of the surgical instrument; and
a surgical camera rotatably coupled to the capsule, the surgical camera rotatable between a closed position, wherein the surgical camera is substantially disposed within the lumen of the capsule, and an open position, wherein the surgical camera is substantially displaced from the lumen of the capsule, the surgical camera translating in conjunction with the capsule between the initial position and the extended position.

8. The surgical camera assembly according to claim 7, wherein the surgical camera is biased toward the closed position.

9. The surgical camera assembly according to claim 7 or claim 8, wherein the capsule is biased toward the retracted position.

10. The surgical camera assembly according to any of claims 7 to 9, wherein the surgical camera is rotated from the closed position to the open position upon passage of the surgical instrument from the lumen of the elongated tubular member into the lumen of the capsule.

11. The surgical camera assembly according to any of claims 7 to 10, wherein the capsule includes a pick-up element disposed at a distal end thereof and extending inwardly into the lumen of the capsule to dock the capsule on the surgical instrument upon passage of the surgical instrument at least partially through the lumen of the capsule.

12. The surgical camera assembly according to any of claims 7 to 11, wherein the capsule further includes at least one cleaning element, the at least one cleaning element configured to clean a lens of the surgical camera upon movement of the surgical camera from the closed position to the open position.

13. The surgical camera assembly according to any of claims 7 to 12, further comprising a seal member disposed within the lumen of the elongated tubular member, the seal member configured to inhibit the escape of fluid through the lumen of the elongated tubular member.

14. The surgical camera assembly according to claim 7 to 13, wherein the surgical camera is hingably coupled to the capsule via at least one hinge, the surgical camera rotatable about the at least one hinge between the open and closed positions.

15. The surgical camera assembly according to any of claims 7 to 14, wherein the capsule is releasably coupled to the elongated tubular member.
